# EUROPEAN PATENT SPECIFICATION

(11) **EP 1 439 357 B1**
(45) Date of publication and mention of the grant of the patent: **23.05.2007**
(21) Application number: 03015897.6
(22) Date of filing: 11.07.2003
(51) Int. Cl.: F25D 23/06, B32B 37/00, A61L 2/23, F25B 47/00

(54) **Refrigerator with an inner case containing nanosilver particles**
Kühlschrank mit einem Silberteilchen im Nanobereich aufweisenden Innenbehälter
Réfrigérateur avec un boîtier interne contenant des nanoparticules d'argent

(30) Priority: 14.01.2003 KR 2003002294
(43) Date of publication of application: 21.07.2004
(73) Proprietor: Daewoo Electronics Corporation, Seoul (KR)
(72) Inventor: Jin, Soo Kim, Daewoo Electronics Corporation, Seoul (KR)
(74) Representative: Turi, Michael

(56) References cited:
- EP-A- 0 270 129
- WO-A-00/64259
- DE-A1- 10 120 802
- DE-U1- 20 214 601
- PATENT ABSTRACTS OF JAPAN vol. 1996, no. 12, 26 December 1996 (1996-12-26) & JP 08 210761 A (MATSUSHITA REFRIG CO LTD), 20 August 1996 (1996-08-20)
- PATENT ABSTRACTS OF JAPAN vol. 018, no. 328 (C-1215), 22 June 1994 (1994-06-22) & JP 06 073196 A (HITACHI LTD), 15 March 1994 (1994-03-15)

## Description

The present invention relates to a refrigerator with an inner case for defining an inner space of the refrigerator; and, more particularly, to a refrigerator including an inner case divided into two parts one of which contains nanosilver particles exhibiting optimal antibacterial and/or antifungal functions.

In general, silver (Ag) is well-known as a common antibacterial agent. In particular, colloidal silver is known as being safe and effective against, bacteria, fungi, microbes, virus and the like. When silver ions are absorbed into cells of virus, bacteria, fungi and the like, the silver ions prevent the growth of enzyme required in respiration thereof to render them dead. Further, silver ions prevent metabolism of the bacteria and suppress reproduction thereof.

Fine particles of silver can be produced by a physical process such as electrolysis, liquid phase reduction, and grinding. The electrolysis has mainly been used hitherto in order to obtain stabilized nanosilver particles with a high purity. In the electrolysis process, pure silver (99.99%) is immersed into distilled water; and extremely fine particulates of silver are obtained by applying a low current at a low temperature.

Meanwhile, a refrigerator serves to preserve food in a fresh state for an extended period of time in a food storage compartment by cooling air in the compartment using a refrigeration cycle. In the refrigerator, refrigerant gas compressed under a high temperature and a high pressure by a compressor is condensed into liquid phase in a condenser; and the liquid is then pressure-reduced through an expansion valve and evaporated in an evaporator. At this time, the evaporating refrigerant takes heat from the ambient air to cool it. The cooled air is then forced into the food storage compartment by, e.g., a fan.

A study on employing the above-described nanosilver for an antibacterial purpose in the refrigerator has been conducted, in which the inner case of the refrigerator, which defines an inner space of the refrigerator, serves the antibacterial purpose by making it of nanosilver.

The inner case of the refrigerator is typically made of acrylonitrile butadiene styrene("ABS") copolymer since the styrene base resin such as the ABS copolymer has an excellent mechanical property, workability, appearance, gloss and the like. The inner case of the refrigerator is manufactured by the ABS copolymer to a sheet and then performing a vacuum forming process on it.

The vacuum forming process is one type of plastic forming process, the vacuum forming process including extruding a resin to a sheet, heating and softening the sheet in a vacuum forming machine to form a softened sheet, and drawing the softened sheet to a mold so as to form it into a shape required by using a vacuum of air or a vacuum and a compact of air to manufacture a desired product. This vacuum forming process is widely used to produce the inner case of the refrigerator.

Such an inner case is mixed with nanosilver particles by adding nanosilver particles to the ABS solvent contained in the master batch. This permits nanosilver to be wastefully used since nanosilver particles are added even in forming a portion of the inner case in which antifungal function is not required. Accordingly, such production is cost ineffective.

DE 202 14 601 U1 discloses an inner portion for a cooling device, wherein the inner portion is provided with an inner layer having silver particles embedded therein.

EP 0 270 129 A2 discloses antibiotic zeolite including silver particles having an average particle size of 1500 nm.

DE 101 20 802 A1 discloses a method for fabrication of coated nanoparticles.

WO 00/64259 discloses a cooler chest with antimicrobial properties.

In this specification, the term "nanosilver particles" indicates silver particles being nano-sized. This also applies to the claims.

It is, therefore, a primary object of the present invention to provide a refrigerator including an inner case capable of having reduced amount of nanosilver particles to be contained therein.

In accordance with an aspect of the present invention, there is provided a refrigerator including: an inner case having an inner sheet for defining an inner space of the refrigerator and an outer sheet formed on the inner sheet, the inner sheet containing nanosilver particles therein, wherein the nanosilver particles have a size of about 15 ~ 300 nm and a concentration of about 1 ~ 500 ppm by weight.

Preferably, the inner sheet and the outer sheet are bonded to each other by a double extrusion molding technique.

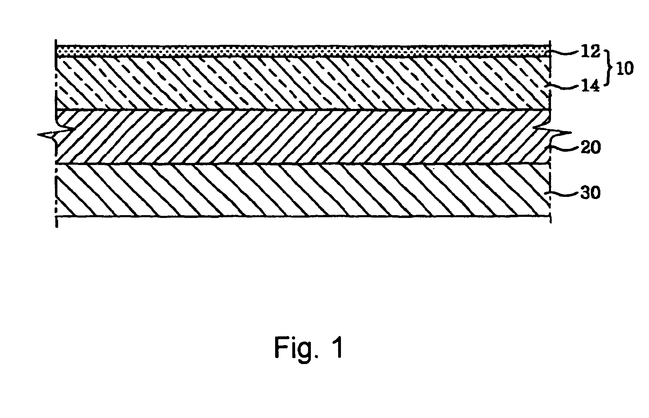

The above and other objects and features of the present invention will become apparent from the following description of preferred embodiments given in conjunction with the accompanying drawing in which:

The drawing is a schematic cross sectional view of an inner case of a refrigerator in accordance with the present invention.

A preferred embodiment of the present invention will now be described in detail with reference to a drawing showing a schematic cross sectional view of a case of a refrigerator in accordance with the present invention.

As shown, the case includes an inner case 10, an outer case 30 for forming an appearance of the refrigerator and a heat insulating material 20 provided therebetween.

The inner case 10 has an inner sheet 12 for defining an inner space of the refrigerator and an outer sheet 14 formed on the heat insulating material 20. The inner sheet 12 may come into contact with food, the user or the air contaminants and thus the antifungal function is required, while the outer sheet 14 does not. Accordingly, nanosilver particles having antifungal function are contained in the only inner sheet 12 while the outer sheet 14 is made of a typical acrylonitrile butadiene styrene("ABS") copolymer. This reduces amount of the nanosilver particles to be used in the inner case 10. Accordingly, it is preferable that the thickness of the inner sheet 12 is minimized so as to further decrease amount of nanosilver to be contained therein. The inner sheet 12 and the outer sheet 14 are bonded to each other by, e.g., a double extrusion molding technique.

In the preferred embodiment of the present invention, the size of the nanosilver particles is limited to about 15-300 nm in consideration of the current manufacturing technique and the efficiency of specific surface area per unit weight.

Further, the concentration of the nanosilver added to the inner sheet 12 is limited to a range of about 1-500 ppm by weight such that the expensive nanosilver can efficiently be used and a target antibacterial ability can be obtained. As the concentration of the nanosilver is increased, the antibacterial ability is enhanced; however, it is found that, in the range of 50-500 ppm by weight, there is no significant difference in the antibacterial ability. In addition, in the above concentration range of nanosilver, the sterilization or bacteriostasis can be performed stably and efficiently.

With such an inner case of the refrigerator, it is possible to reduce the product cost of the refrigerator since amount of nanosilver particles to be contained in the inner case thereof is reduced.

## Claims

1. A refrigerator comprising :
an inner case (10) including an inner sheet (12) for defining an inner space of the refrigerator and an outer sheet (14) formed on the inner sheet (12), the inner sheet containing nanosilver particles therein, **characterized in that** the nanosilver particles have a size of 15∼300 nm and a concentration of about 1∼500 ppm by weight.

2. The refrigerator of claim 1, wherein the inner sheet (12) and the outer sheet (14) are bonded to each other by a double extrusion molding technique.

## Patentansprüche

1. Kühlschrank mit:
einem inneren Gehäuse (10) mit einer inneren Lage (12) zum Definieren eines Innenraums des Kühlschranks und einer äußeren Lage (14), die auf der inneren Lage (12) ausgebildet ist, wobei die innere Lage darin Silberpartikel in Nanogröße enthält, **dadurch gekennzeichnet, dass** die Silberpartikel in Nanogröße eine Größe von 15 ∼ 300 nm und eine Konzentration von etwa 1 ~ 500 ppm pro Gewicht haben.

2. Kühlschrank nach Anspruch 1, bei dem die innere Lage (12) und die äußere Lage (14) mittels eines doppelten Extrusionsformverfahrens miteinander verbunden sind.

## Revendications

1. Réfrigérateur comprenant :
un boîtier interne (10) comportant une feuille interne (12) pour définir un espace intérieur du réfrigérateur et une feuille externe (14) formée sur la feuille interne (12), la feuille interne contenant des nanoparticules d'argent, **caractérisé en ce que** les nanoparticules d'argent ont une taille de 15∼300 nm et une concentration en poids d'environ 1∼500 ppm.

2. Réfrigérateur selon la revendication 1, dans lequel la feuille interne (12) et la feuille externe (14) sont liées ensemble par une technique de moulage par double extrusion.
